# EUROPÄISCHE PATENTSCHRIFT

(11) **EP 0 445 387 B1**
(45) Veröffentlichungstag und Bekanntmachung des Hinweises auf die Patenterteilung: **08.06.1994**
(21) Anmeldenummer: 90123510.1
(22) Anmeldetag: 07.12.1990
(51) Int. Cl.: A61M 5/142, F04B 43/08

(54) **Lineare Schlauchpumpe**
Linear hose pump
Pompe linéaire à tuyau

(30) Priorität: 03.03.1990 DE 4006763
(43) Veröffentlichungstag der Anmeldung: 11.09.1991
(73) Patentinhaber: B. Braun Melsungen AG, 34209 Melsungen (DE)
(72) Erfinder: Gerlach, Hans, W-3538 Marsberg 4 (DE); Knuth, Reinhard, W-3508 Melsungen (DE)
(74) Vertreter: Selting, Günther, Dipl.-Ing.

(56) Entgegenhaltungen:
- EP-A- 0 283 614
- DE-A- 3 227 051
- FR-A- 1 470 136
- US-A- 3 489 097
- US-A- 3 778 195

## Beschreibung

Die Erfindung betrifft eine lineare Schlauchpumpe der im Oberbegriff des Patentanspruchs 1 angegebenen Art.

Im medizinischen Bereich werden häufig lineare Schlauchpumpen als Infusionspumpen verwendet, um Flüssigkeit aus einem Vorratsbehälter dem Körper eines Patienten zuzuführen. Die Schlauchpumpe weist einen Schlauch auf, der als preisgünstiger, steriler Einmalartikel in das Pumpengehäuse eingesetzt und mit der ankommenden und der abgehenden Schlauchleitung verbunden wird. Dieser Schlauch wird durch eine Quetschvorrichtung abgedrückt, die zahlreiche, von einer Nockenwelle angetriebene Pumpenfinger aufweist, welche zyklisch in unterschiedlichen Phasenlagen auf den Pumpenschlauch einwirken und diesen abquetschen. Der Schlauch besitzt eine Rückstellfähigkeit, so daß er sich bei einem Rückzug eines Pumpenfingers an dieser Stelle wieder aufweitet.

Der Prospekt INFUSOMAT secura der Firma B. Braun Melsungen AG beschreibt eine Infusionsvorrichtung, die eine lineare Schlauchpumpe enthält. Die Quetschvorrichtung, die mit zahlreichen Pumpenfingern auf den Schlauch einwirkt, befindet sich im Gehäuseinneren und der Schlauch wird von der entgegengesetzten Seite durch ein federndes Gegenlager abgestützt, das an einer Gehäusetür angebracht ist. Das federnde Gegenlager dient zur Anpassung an die Schlauchtoleranzen. Die auswechselbar in die Schlauchpumpe einsetzbaren Schläuche haben sowohl hinsichtlich ihrer Querschnittsabmessungen als auch hinsichtlich ihrer Shorehärte gewisse Fertigungstoleranzen. Aus diesem Grund ist eine nachgiebige Andrückvorrichtung erforderlich, die das Gegenlager in Richtung auf die Quetschvorrichtung drückt. Durch die Andrückvorrichtung wird unter anderem sichergestellt, daß die Pumpe den erforderlichen Förderdruck aufbaut, daß aber andererseits der Schlauchinnendruck die Berstfestigkeit der mit dem Schlauch verbundenen Infusionsleitung nicht überschreitet. Im allgemeinen beträgt die maximale Förderratenabweichung linearer Schlauchpumpen +/- 5 % und der erzeugte Förderdruck liegt in der Größenordnung von 1,5 bar. Das federnd an der Gehäusetür abgestützte Gegenlager der bekannten Schlauchpumpe verursacht eine Reihe von Nachteilen. Die an der Gehäusetür abgestützten Federn haben eine kurze Baulänge, damit die Gesamtheit aus Gehäusetür und Gegenlager eine geringe Dicke hat. Es werden daher kurze Federn mit steiler Federkennlinie benutzt, welche die Toleranzen nur begrenzt auffangen können. Die Toleranzen der Schlauchpumpe und ihres Einbaus in das Gehäuse müssen sehr eng gehalten werden, um die gewünschte Andruckkraft zwischen den Pumpenfingern und dem Gegenlager zu erhalten. Im Falle größerer Toleranzen muß die Federkraft verstellbar sein, was zusätzliche Justiermittel an der Gehäusetür erfordert. Ferner ist das Gegenlager an der Gehäusetür mit mehreren Federn abgestützt. Durch die wechselnde Belastung der Pumpenfinger ergeben sich unterschiedliche Federkräfte und damit eine wechselnde Belastung der Lager der Nockenwelle des Antriebsmotors sowie die Gefahr der Schrägstellung des Gegenlagers, mit der Folge einer Veränderung der Spaltgröße. Schließlich ist das Gegenlager an der Gehäusetür angebracht, die geöffnet werden kann. Es ist daher der Gefahr der Verschmutzung ausgesetzt.

In der dem Oberbegriff des Anspruchs 1 zugrundeliegenden EP 0 283 614 ist eine lineare Schlauchpumpe beschrieben, bei der das Widerlager fest an einem Führungsgehäuse für die Pumpenfinger angeordnet ist. An diesem Führungsgehäuse ist das die Nockenwelle für die Pumpenfinger enthaltende Gehäuse schwenkbar angebracht und eine Federvorrichtung schwenkt das Nockenwellengehäuse so, daß die Nockenwelle mit den Pumpenfedern in Richtung auf das feste Gegenlager gedrückt wird. Diese Lösung hat jedoch den Nachteil, daß ein zweiteiliges Pumpengehäuse erforderlich ist, dessen Teile durch eine Gelenkachse miteinander verbunden sind, und daß eine exakte gegenseitige Führung der beiden Gehäuseteile nur schwer zu erreichen ist. Die Andrückvorrichtung ist nicht justierbar und muß aus mindestens zwei separaten Federn bestehen.

Der Erfindung liegt die Aufgabe zugrunde, eine lineare Schlauchpumpe der im Oberbegriff des Patentanspruchs 1 angegebenen Art zu schaffen, bei der die die Quetschvorrichtung und das Gegenlager gegeneinanderdrückende Andrückvorrichtung keinen konstruktionsbedingten Beschränkungen unterliegt, so daß die Anpassung an unterschiedliche Toleranzen der Pumpenkonstruktion und des Schlauchs und an verschleißbedingte Änderungen in weiten Grenzen mit einfachen konstruktiven Mitteln ermöglicht wird.

Die Lösung dieser Aufgabe erfolgt erfindungsgemäß mit den im Patentanspruch 1 angegebenen Merkmalen.

Bei der erfindungsgemäßen Schlauchpumpe ist die Quetschvorrichtung in einem Pumpengehäuse untergebracht, welches von einem Rahmen übergriffen wird, an dem das Gegenlager angebracht ist. Das Pumpengehäuse mit der Quetschvorrichtung ist relativ zu dem Rahmen in Richtung auf das Gegenlager verschiebbar geführt und die Andrückvorrichtung ist an dem dem Gegenlager abgewandten Ende des Rahmens abgestützt und sie drückt gegen die Quetschvorrichtung. Die Andrückvorrichtung greift somit an der dem Gegenlager abgewandten Rückseite der Quetschvorrichtung an. Sie unterliegt hinsichtlich ihrer baulichen Gestaltung keinen Einschränkungen, weil an der Rückseite des Pumpengehäuses normalerweise hinreichend Platz zur Verfügung steht. Die Andrückvorrichtung kann daher eine relativ große Länge haben, mit der Folge eines großen Federweges. In der Andrückvorrichtung kann daher eine Feder (oder mehrere Federn) verwendet werden, die relativ weich ist, d.h. eine geringe Federkonstante aufweist und in dem zu beherrschenden Toleranzbereich eine nahezu konstante, d.h. weitgehend wegunabhängige, Andrückkraft liefern. Ferner steht auf der Rückseite der Quetschvorrichtung hinreichend Platz für eine Verstellvorrichtung zum Verstellen der Kraft der Andrückvorrichtung zur Verfügung.

Vorzugsweise ist eine einzige Andrückvorrichtung vorgesehen, die zentral auf die Quetschvorrichtung einwirkt, wobei die Quetschvorrichtung an einer Linearführung des Rahmens geführt ist.

Die erfindungsgemäße Schlauchpumpe besteht aus lediglich drei Baugruppen, nämlich der Quetschvorrichtung, die in einem völlig geschlossenen Pumpengehäuse untergebracht sein kann, dem Rahmen, an dem das Gegenlager angebracht ist, und der Andrückvorrichtung, die an dem Rahmen abgestützt ist und die die Quetschvorrichtung in Richtung auf das Gegenlager treibt. Die Schlauchpumpe ist von einfachem Aufbau und von robuster Ausführung. Diejenigen Teile, die der Umgebung ausgesetzt sind und verschmutzen können, sind von einfachem Aufbau, so daß sie leicht gereinigt und desinfiziert werden können. Am Gegenlager sind keine Federn oder andere Einrichtungen, die einer leichten Verschmutzung unterliegen, angebracht.

Die Schlauchpumpe ist vorzugsweise in dem Gehäuse eines Infusionsgerätes angebracht, das außerdem noch eine Eingabeeinrichtung sowie eine Steuereinrichtung für den Schlauchpumpenbetrieb enthält. Das Gegenlager ist dabei als aufklappbare Tür an der Außenseite der Gehäusewand angeordnet. Im geschlossenen und verriegelten Zustand der Tür ist die Tür starrer Bestandteil des Rahmens, der sich durch die Gehäusewand in das Gehäuseinnere hinein erstreckt.

Im folgenden werden unter Bezugnahme auf die Zeichnungen Ausführungsbeispiele der Erfindung näher erläutert.

Es zeigen:

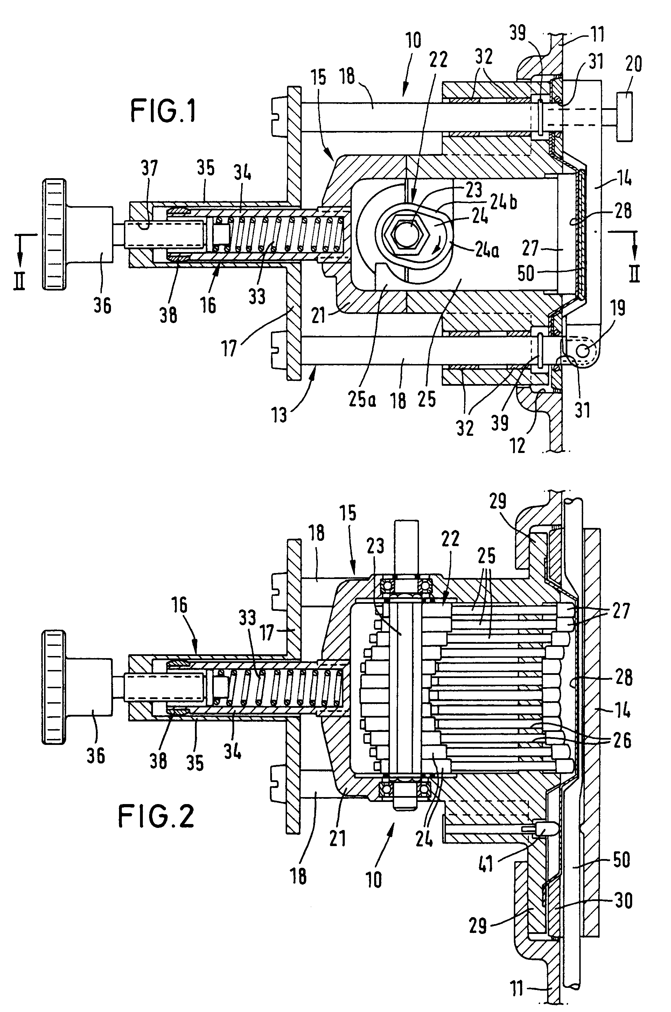

- Fig. 1: einen Horizontalschnitt durch die Schlauchpumpe,
- Fig. 2: einen Vertikalschnitt entlang der Linie II-II von Fig. 1 und
- Fig. 3: in ähnlicher Darstellung wie Fig. 1 eine zweite Ausführungsform der Schlauchpumpe.

Die Schlauchpumpe 10 ist in einer frontseitigen Gehäusewand 11 des Gehäuses eines Infusionsgerätes in einer Aussparung 12 montiert. Sie besteht aus dem starren Rahmen 13 mit dem Gegenlager 14, der Quetschvorrichtung 15 und der Andrückvorrichtung 16. Der Rahmen 13 weist ein Basisteil 17 und vier davon abstehende Stangen 18 auf. An zwei der Stangen 18 ist über ein Gelenk 19 das Gegenlager 14 angebracht, das als aufklappbare Tür ausgebildet ist und an den beiden anderen Stangen 18 mit einem Schnellverschluß 20 befestigt werden kann.

Ein (nicht dargestellter) Antriebsmotor, der an dem Pumpengehäuse 21 befestigt ist, treibt über ein Untersetzungsgetriebe die in dem Pumpengehäuse 21 gelagerte Nockenwelle 22. Die Nockenwelle 22 besteht aus einer Welle 23 mit Sechskantprofil und auf dieser Welle sitzenden Nockenscheiben 24, die jeweils eine Sechskantöffnung aufweisen und dadurch drehfest auf der Welle 23 sitzen. Je zwei Nockenscheiben 24 bilden eine Doppelnockenscheibe. Benachbarte Nockenscheiben sind umfangsmäßig um 30° zueinander versetzt, auf der Welle 23 angeordnet und haben eine Exzentrizität von 2,5 mm, wodurch sich bei einer vollen Umdrehung der Nockenscheibe ein Hub von 5 mm ergibt.

Jede der Nockenscheiben 24 treibt einen Pumpenfinger 25. Die Pumpenfinger sind in dem Pumpengehäuse 21 in seitlichen Nuten längsverschiebbar geführt und sie verlaufen durch Schlitze 26 der dem Gegenlager 14 zugewandten Wand des Pumpengehäuses hindurch, wobei am Ende eines jeden Pumpenfingers 25 ein verdickter Kopf 27 angeordnet ist. Das Lagerspiel der Führungsnuten für die Pumpenfinger 25 ist so gewählt, daß sich die Köpfe benachbarter Pumpenfinger gerade berühren, daß sich jedoch Toleranzen der Pumpenfinger bzw. der Köpfe nicht addieren.

Die Köpfe 27 der Pumpenfinger sind von einer Membran 28 bedeckt, die an dem Pumpengehäuse 21 befestigt ist. Das Pumpengehäuse 21 weist ferner Flansche 29 auf, mit denen es an der Gehäusewand 11 des Gerätegehäuses befestigt ist. Ein Blendrahmen 30, der bündig und abdichtend in die Öffnung 12 der Gehäusewand 11 eingesetzt ist, bildet den äußeren Abschluß des Pumpengehäuses. Dieser Blendrahmen, der die Membran 28 umschließt und einspannt, enthält Durchtrittsöffnungen 31 mit Dichtungen für den Durchtritt der Stangen 18.

Das Pumpengehäuse 21 ist mit Gleitbuchsen 32 längs der Stangen 18 geführt, so daß der Rahmen 13 relativ zu dem Pumpengehäuse 21 bzw. zur Quetschvorrichtung 15 in Längsrichtung der Stangen 18 bewegt werden kann. Wenn das Gegenlager 14 sich in der Schließstellung befindet, ist es starrer Bestandteil des Rahmens 13 und kann mit diesem relativ zur Gehäusewand 11 bzw. zu der darin befestigten Quetschvorrichtung 15 horizontal verschoben werden.

An der Rückseite des Pumpengehäuses 21 ist eine aus mindestens einer Druckfeder bestehende Federvorrichtung 33 abgestützt. Die Federvorrichtung 33 ist in einem mit dem Pumpengehäuse 21 verbundenen Rohr 34 enthalten, das teleskopisch in ein von dem Basisteil 17 nach hinten abstehendes Rohr 35 eintaucht. Am Ende des Rohres 35 befindet sich eine Stellvorrichtung 36, die mit einem Gewinde in ein Innengewinde 37 des Rohres 35 eingreift und mit ihrem Ende gegen die Federvorrichtung 33 drückt. Durch Drehen der Stellvorrichtung 36 kann die Federkraft der Andrückvorrichtung 16 verändert werden. Die Federvorrichtung 33 greift an dem Basisteil 17 und an der Quetschvorrichtung 15 zentrisch an, d.h. entlang der Mittelachse der Quetschvorrichtung. Am Rohr 34 ist ein Gleitstück 38 vorgesehen, das ein reibungsarmes Gleiten der beiden Rohre 34 und 35 ermöglicht und das zusammen mit den Gleitbuchsen 32 dafür sorgt, daß der Rahmen 13 in Bezug auf die Quetschvorrichtung 15 verkantungsfrei linear verschiebbar geführt ist. Die Bewegung des Rahmens 13 relativ zu der Quetschvorrichtung 15 wird durch Flansche 39 begrenzt, die an den Stangen 18 vorgesehen sind.

Die Form der Nockenscheiben 24 ist generell kreisförmig, wobei jede Nockenscheibe exzentrisch auf der Welle 23 angeordnet ist. Bei dem vorliegenden Ausführungsbeispiel weicht die Außenkontur der Nockenscheiben 24 jedoch, wie nachstehend erläutert wird, von der Kreisform ab. Die Nockenscheiben 24 haben an der Stelle des größten Hubes eine Abflachung 24a von 0,15 mm. Hierdurch wird die an sich sinusförmige Wellenbewegung in ihrem Maximum linearisiert. Der Pumpenfinger verharrt über einen Drehwinkel von ca. 34° in seiner vorderen Endstellung. In dieser Zeit wird der zwischen der Membran 28 und dem Gegenlager 14 vertikal verlaufende Pumpenschlauch 50 okklusiv gedrückt, jedoch nicht überquetscht. Während ein Pumpenfinger sich noch in der maximalen Vorschubstellung befindet, gelangt bereits der nachfolgende Pumpenfinger ebenfalls in die maximale Vorschubstellung und übernimmt somit das Abquetschen des Schlauchs 50. Da sich die maximalen Vorschubstellungen benachbarter Pumpenfinger zeitlich überlappen, wird eine Rückförderung vermieden. Die untere Hubbegrenzung (in der Rückzugsstellung der Pumpenfinger) erfolgt dadurch, daß der Pumpenfinger gegen einen Anschlag am Pumpengehäuse stößt. Diese Maßnahmen führen zu einem weitgehend linearen Förderverhalten der Pumpe. Die Anschläge, die die Rückwärtsbewegungen der Pumpenfinger 25 begrenzen, sind so angeordnet, daß der Pumpenschlauch auch bei zurückgezogenem Pumpenfinger noch ein wenig im Sinne einer Zusammendrückung deformiert gehalten wird und somit stets geringfügig vorgespannt ist. Hierdurch wird die Fördergenauigkeit bei schwankendem einlaufseitigem Druck erhöht.

Durch die oben geschilderten Maßnahmen wird der Gesamthub der Pumpenfinger jeweils auf 3 mm begrenzt, wodurch erreicht wird, daß ein Pumpenschlauch mit einem Innendurchmesser von 4 mm stets auf der gesamten Schlauchlänge an irgendeiner Stelle zusammengedrückt wird. Hierdurch wird erreicht, daß ein Vordruck am Eingang des Pumpenschlauchs sich nicht auf die Förderrate auswirkt.

Der Umfang der Nockenscheiben 24 ist an der dem Bereich 24a folgenden Rückflanke mit einer Abflachung 24b versehen, um bei jeder Zurückbewegung die Rückzugszeit zu verkürzen. Um die Pumpenfinger oder Pumpenschieber in die hintere Stellung zurückzuziehen, ist jeder Pumpenfinger 25 mit einer angeformten Nase 25a versehen, unter die der entsprechende Nocken 24b greift und aus der sich dieser Nocken herausdreht, wenn der Pumpenfinger gegen seinen rückwärtigen Anschlag gestoßen ist. Durch die beschriebene Anordnung von Nockenscheiben 24 und Pumpenfingern 25 ist gewährleistet, daß jeweils mindestens ein Nockenfinger sich jeweils in der vordersten Position befindet.

Im Betrieb wird der Schlauch 50, der zwischen der Membran 28 und dem Gegenlager 14 vertikal verläuft, in jeder Phase der Umdrehung der Nockenwelle 22 durch mindestens einen Pumpenfinger 25 okklusiv abgequetscht. Etwaige Toleranzen von Pumpe und/oder Schlauch werden durch die Andrückvorrichtung 16 ausgeglichen, die durch die Federvorrichtung 33 bewirkt, daß der Rahmen 13 zusammen mit dem Gegenlager 14 gegen die Quetschvorrichtung 15, und somit auch gegen die Pumpenfinger 25 getrieben wird. Bei einem im Schlauch vorhandenen Gegendruck versucht dieser Gegendruck, den Pumpenschlauch aufzuweiten. Diese Aufweitungstendenz bewirkt ein Zusammendrücken der Federvorrichtung 33.

Auf der Auslaßseite der Schlauchpumpe ist am Pumpengehäuse 21 ein Druckschieber 41 verschiebbar angeordnet, der gegen die Membran 28 und gegen den Auslaßbereich des Pumpenschlauchs drückt. Der Schieber 41 ist mit einem (nicht dargestellten) Sensor gekoppelt, der die Stellung des Schiebers 41 feststellt und dadurch den Ausgangsdruck der Pumpe ermittelt.

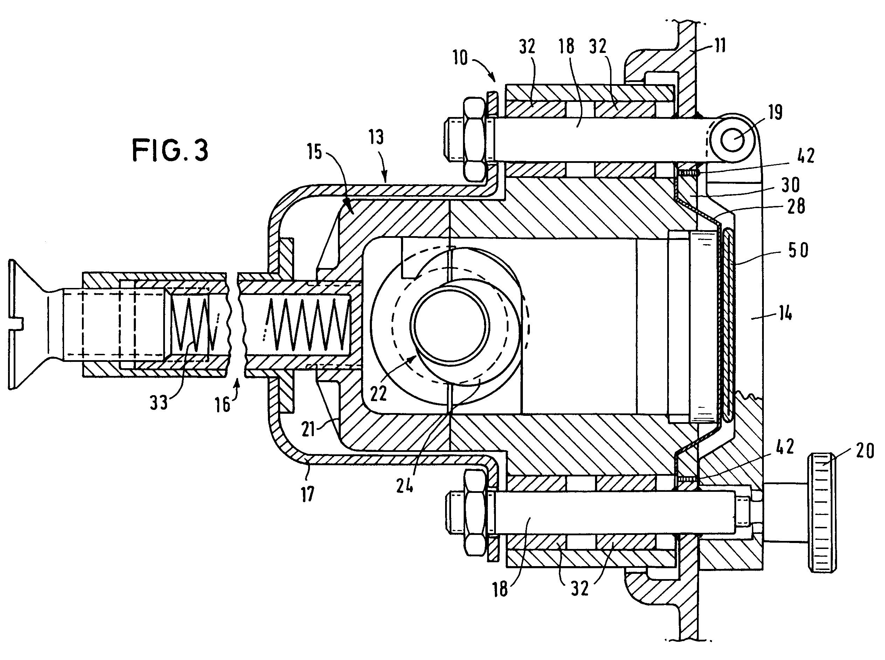

Das Ausführungsbeispiel von Fig. 3 unterscheidet sich von dem ersten Ausführungsbeispiel im wesentlichen dadurch, daß der Rahmen 13 in Bezug auf die Gehäusewand 11 fest angebracht ist, während die Quetschvorrichtung 15 relativ zu dem Rahmen 13 und zu der Gehäusewand 11 verschiebbar ist. Die Stangen 18 des Rahmens sind fest mit der Gehäusewand 11 verbunden, so daß auch das die Tür bildende Gegenlager 14 an ortsfest zu der Gehäusewand angeordneten Gelenken 19 schwenkbar ist. Die Blende 30 ist mit dem Pumpengehäuse 21 verbunden, wobei der Rand der Membran 28 zwischen Pumpengehäuse 21 und Blende 30 eingespannt ist. Zwischen Blende 30 und Gehäusewand 11 befindet sich eine Dichtung 42, die Verschiebungen der Quetschvorrichtung in Bezug auf die Gehäusewand zuläßt.

Bei dem Ausführungsbeispiel von Fig. 3 ist das Basisteil 17 ein U-förmiger Bügel, der die Quetschvorrichtung auf einem Teil ihrer Länge umgibt, so daß die Stangen 18 des Rahmens 13 kürzer ausgebildet sein können als bei dem ersten Ausführungsbeispiel. Die Andrückvorrichtung 16 drückt die Quetschvorrichtung 15 in Richtung auf das Gegenlager 14.

## Patentansprüche

1. Lineare Schlauchpumpe mit einer einen Schlauch (50) in Längsrichtung fortlaufend zusammendrückenden Quetschvorrichtung (15), einem den Schlauch abstützenden Gegenlager (14) und einer nachgiebigen Andrückvorrichtung (16), die die Quetschvorrichtung (15) und das Gegenlager (14) gegeneinanderdrückt,
**dadurch gekennzeichnet**,
daß die Quetschvorrichtung (15) von einem einteiligen starren Pumpengehäuse (21) umgeben ist, daß das Gegenlager (14) mit einem das Pumpengehäuse (21) übergreifenden Rahmen (13) verbunden ist, daß das Pumpengehäuse (21) und der Rahmen (13) relativ zueinander verschiebbar geführt sind und daß die Andrückvorrichtung (16) an dem Rahmen (13) abgestützt ist und gegen das dem Gegenlager (14) abgewandte Ende des Pumpengehäuses (21) drückt.

2. Schlauchpumpe nach Anspruch 1, dadurch gekennzeichnet, daß das Pumpengehäuse (21) hinter einer Gehäusewand (11) befestigt ist, daß Stangen (18) des Rahmens (13) verschiebbar durch die Gehäusewand (11) hindurchragen und daß das Gegenlager (14) vor der Gehäusewand als Deckel angeordnet ist.

3. Schlauchpumpe nach Anspruch 1, dadurch gekennzeichnet, daß der Rahmen (13) an einer Gehäusewand (11) befestigt ist und daß an dieser Gehäusewand das Gegenlager (14) als Deckel angeordnet ist.

4. Schlauchpumpe nach einem der Ansprüche 1-3, dadurch gekennzeichnet, daß die Andrückvorrichtung (16) eine einzige mittig auf die Rückseite der Quetschvorrichtung (15) einwirkende Federvorrichtung (33) aufweist.

5. Schlauchpumpe nach einem der Ansprüche 1-4, dadurch gekennzeichnet, daß Stangen (18) des Rahmens (13) eine Linearführung für an dem Pumpengehäuse (21) vorgesehen Buchsen (32) bilden.

6. Schlauchpumpe nach einem der Ansprüche 1-5, dadurch gekennzeichnet, daß die Quetschvorrichtung (15) eine Nockenwelle (22) mit Nockenscheiben (24) aufweist, daß die Nockenscheiben (24) jeweils einen Pumpenfinger (25) steuern und daß jede Nockenscheibe (24) an der Stelle des größten Hubes des zugehörigen Pumpenfingers (25) eine Abflachung (24a) aufweist.

7. Schlauchpumpe nach Anspruch 6, dadurch gekennzeichnet, daß jeder Pumpenfinger (25) eine Nase (25a) aufweist, die zum Rückziehen dieses Pumpenfingers von der zugehörigen Nockenscheibe (24) untergriffen wird und die die Nockenscheibe (24) freigibt, nachdem diese Nockenscheibe gegen einen Anschlag des Pumpengehäuses (21) gestoßen ist.

## Claims

1. A linear hose pump, comprising a squeezing device (15) progressively squeezing a hose (50) in longitudinal direction, a thrust bearing (14) supporting the hose, and a flexible pressing device (16) pressing the squeezing device (15) and the thrust bearing (14) against each other,
**characterized in**
that the squeezing device (15) is surrounded by a one-piece rigid pump housing (21), that the thrust bearing (14) is connected with a frame (13) covering the pump housing (21), that the pump housing (21) and the frame (13) are guided to be displaceable relative to each other, and that the pressing device (16) is supported on the frame (13) and presses against that end of the pump housing (21), which faces away from the thrust bearing (14).

2. The hose pump according to claim 1, characterized in that the pump housing (21) is fastened behind a housing wall (11), that rods (18) of the frame (13) displaceably protrude through the housing wall (11), and that the thrust bearing (14) is arranged as a cover in front of the housing wall.

3. The hose pump according to claim 1, characterized in that the frame (13) is fastened to a housing wall (11), and that the thrust bearing (14) is arranged as a cover at this housing wall.

4. The hose pump according to one of claims 1-3, characterized in that the pressing device (16) comprises a single spring device (33) acting centrally upon the backside of the squeezing device (15).

5. The hose pump according to one of claims 1-4, characterized in that rods (18) of the frame (13) form a linear guide for bushings (32) provided at the pump housing (21).

6. The hose pump according to one of claims 1-5, characterized in that the squeezing device (15) comprises a camshaft (22) with cam disks (24), that each of the cam disks (24) controls a pump finger (25), and that each cam disk (24) comprises a flattening (24a) at the position of the largest stroke of the associated pump finger (25).

7. The hose pump according to claim 7, characterized in that each pump finger (25) comprises a catch (25a) being engaged from below by the associated cam disk (24) in order to retract this pump finger, and releasing the cam disk (24) after this cam disk (24) has abutted a stop of the pump housing (21).

## Revendications

1. Pompe à tuyau souple linéaire comportant un dispositif de pincement (15), qui comprime progressivement un tuyau souple (50) dans la direction longitudinale, une contre-butée (14) supportant le tuyau souple et un dispositif de poussée (16) élastique qui pousse l'un contre l'autre le dispositif de pincement (15) et la contre-butée (14), caractérisée en ce que le dispositif de pincement (15) est entouré par un carter de pompe rigide, monobloc (21), que la contre-butée (14) est reliée à un cadre (13) qui s'engage par dessus le carter de pompe (21), que le carter de pompe (21) et le cadre (13) sont guidés de manière à être déplaçables l'un par rapport à l'autre, et que le dispositif de poussée (16) est supporté par le cadre (13) et pousse contre l'extrémité du carter de pompe (21), qui est tournée à l'opposé de la contre-butée (14).

2. Pompe à tuyau souple selon la revendication 1, caractérisée en ce que le carter de pompe (21) est fixé derrière une paroi (11) de carter, que des barres (18) du cadre (13) traversent, de manière à y être mobiles, la paroi (11) de carter et que la contre-butée (14) est disposée en tant que couvercle devant la paroi de carter.

3. Pompe à tuyau souple selon la revendication 1, caractérisée en ce que le cadre (13) est fixé à une paroi (11) de carter, et que la contre-butée (14) est disposée en tant que couvercle à cette paroi du carter.

4. Pompe à tuyau souple selon l'une des revendications 1-3, caractérisée en ce que le dispositif de poussée (16) comporte un seul dispositif à ressort (33), qui agit en position centrée sur le côté arrière du dispositif de pincement (15).

5. Pompe à tuyau souple selon l'une des revendications 1-4, caractérisée en ce que les barres (18) du cadre (13) forment un guidage linéaire pour des douilles (32) prévues dans le carter de pompe (21).

6. Pompe à tuyau souple selon l'une des revendications 1-5, caractérisée en ce que le dispositif de pincement (15) possède un arbre à cames (22) comportant des disques de came (24), que les disques de came (24) commandent chacun un doigt de pompe (25), et que chaque disque de came (24) présente un méplat (24a) à l'emplacement de la course maximale du doigt de pompe (25) associé.

7. Pompe à tuyau souple selon la revendication 6, caractérisée en ce que chaque doigt de pompe (25) présente un ergot (25a), au-dessous duquel le disque de came associé (24) s'engage pour rétracter ce doigt de pompe, et qui libère le disque de came (24), après que ce dernier ait rencontré une butée de carter de pompe (21).
